(19) Europäisches Patentamt
European Patent Office
Office européen des brevets

(11) Veröffentlichungsnummer: **0 135 797**
**A2**

(12) # EUROPÄISCHE PATENTANMELDUNG

(21) Anmeldenummer: **84109931.0**

(22) Anmeldetag: **21.08.84**

(51) Int. Cl.⁴: **C 12 P 21/00**, C 12 N 5/00, C 12 N 15/00, C 07 K 15/04, A 61 K 37/02 // (C12P21/00, C12R1:91)

(30) Priorität: **30.08.83 DE 3331108**
**10.05.84 DE 3417277**

(43) Veröffentlichungstag der Anmeldung: **03.04.85**
**Patentblatt 85/14**

(84) Benannte Vertragsstaaten: **AT BE CH DE FR GB IT LI LU NL SE**

(71) Anmelder: **Boehringer Ingelheim International G.m.b.H, D-6507 Ingelheim am Rhein (DE)**

(72) Erfinder: **Adolf, Günther, Dr. Mag., Johannagasse 20/7, A-1050 Wien (AT)**

(54) **Verfahren zur Herstellung von Lymphotoxin und von Lymphotoxin-mRNA.**

(57) Gegenstand der vorliegenden Erfindung ist ein neues Verfahren zur Herstellung von Lymphotoxin und Lymphotoxin-mRNA durch Inkubation von transformierten T-Lymphocyten in permanenter Kultur und anschließende Abtrennung der so gebildeten Zellprodukte.

EP 0 135 797 A2

Case 12/008
Case 12/011
Dr.Fl./Dr.Upp./Kp.

Boehringer Ingelheim
International GmbH

# Verfahren zur Herstellung von Lymphotoxin und von Lymphotoxin-mRNA

Aus der Literatur ist bekannt, daß gewisse Zellen von Säugetieren nach geeigneter Stimulation in vitro in der Lage sind, sogenannte Lymphotoxine zu produzieren (siehe beispielsweise Evans in Cancer Immunol. Immunother. 12, 181-190 (1983) und Granger et al. in Biology of the Lymphokines pp. 141-180, Academic Press 1979). Hierbei stellt Lymphotoxin einer gegebenen Species in vitro kein einheitliches Protein dar, vielmehr werden eine Reihe von Subtypen beschrieben, die sich in Molekulargewicht, Ladung und Stabilität unterscheiden (siehe beispielsweise Granger et al. in Biology of the Lymphokines pp. 141-180, Academic Press 1979). Im humanen System werden beispielsweise vier Klassen von Lymphotoxinen unterschieden, deren Molekulargewichte in den Bereichen von 10.000 bis 20.000 für γLT, 35.000 bis 50.000 für βLT, 70.000 bis 90.000 für αLT und 200.000 bis 600.000 für LT-Komplex liegen.

Lymphotoxine besitzen die Fähigkeit, in vitro

a) die maligne Transformation von Zellen zu verhindern (siehe Evans et al. in Int. J. Cancer 27, 45-49 (1981) und Ransom et al. in J. Natl. Cancer Inst. 69, 741-744 (1982)),

b) cytostatisch oder sogar cytolytisch auf Tumorzellen zu wirken (siehe Evans et al. in Immunopharmacology 3, 347-359 (1981)) und

c) die Empfindlichkeit von Tumorzellen gegenüber der zellulären Immunabwehr des Organismus zu steigern (siehe Evans et al. in Cell. Immunol. 63, 1-15 (1981) und Ransom et al. in Int. J. Cancer 29, 451-458 (1982)).

Desweiteren ist aus der Literatur bekannt, daß Lymphotoxin-Präparationen im Tiermodell auch die Regression von Tumoren herbeiführen können (siehe Khan et al. in Proc. Soc. Exptl. Biol. Med. 169, 291-294 (1982)). Lymphotoxine haben daher große Bedeutung für die Prophylaxe und Therapie maligner Erkrankungen.

Die bisher bekannt gewordenen Verfahren zur Gewinnung von Lymphotoxinen gehen in den meisten Fällen von primären Zellen aus, z.B. von Leukozyten-Präparationen aus peripherem Blut oder Tonsillen des Menschen (siehe Williams et al. in J. Immunol. 130, 518-520 (1983)), von Milzzellen von Mäusen (siehe Aksamit et al. in Infect. Immun. 36, 1028-1035 (1982) und Trivers et al. in J. Immunol. 117, 130-135 (1976)) oder von Leukocyten aus der Bauchhöhle von syrischen Hamstern oder Meerschweinchen (siehe beispielsweise Evans et al. in Int. J. Cancer 27, 45-49 (1981)). Hierbei müssen diese Zellen erst durch Inkubation mit hochmolekularen Mitogenen, z.B. Phytohämagglutinin, zur Produktion von Lymphotoxinen angeregt werden. So konnten auch bisher in den Kulturüberstanden von B-Lymphocyten-Zellinien nur geringe Lymphotoxin-Aktivitäten festgestellt werden (siehe Granger et al. in J. Immunol. 104, 1476 (1970) und Amino et al. in J. Immunol. 113, 1334-1345 (1974)).

Da nach den bisher angewandten Methoden zur Produktion von Lymphotoxinen nur geringe Mengen und in nur geringer Reinheit hergestellt werden konnten, konnten die bisher hergestellten Lymphotoxine weder bis zur Homogenität gereinigt noch in ausreichenden Mengen für umfangreichere Untersuchungen der antineoplastischen Wirkung am Tiermodell zur Verfügung gestellt werden. Darüber hinaus konnte bisher auch noch keine biologisch aktive Lymphotoxin-mRNA nachgewiesen werden; dies ist jedoch die Voraussetzung für die molekulare Klonierung der Lymphotoxin-Gene mittels der Methoden der Gentechnologie.

Der vorliegenden Erfindung lag daher die Aufgabe zugrunde, Lymphotoxin sowie Lymphotoxin-mRNA in ausreichender Menge und Reinheit herzustellen.

Erfindungsgemäß wurde nun gefunden, daß Zellen des sogenannten T-Lymphocyten-Typs von Affen, insbesondere der Gattung Saguinus, beispielsweise die Zellinien L-77/5, A651, A2543, 70N2, 1022 und 1670, vorzugsweise jedoch die Zellinien 1022 und 1670, die durch Infektion mit Viren, vorzugsweise mit Herpesviren wie Herpes virus saimiri oder Herpes virus ateles, in vitro oder in vivo transformiert und zu permantem Wachstum in Kultur befähigt wurden, in einem geeigneten Kulturmedium spontan, das heißt ohne weitere Induktion, erhebliche Mengen an Lymphotoxin und Lymphotoxin-mRNA produzieren.

Ferner wurde gefunden, daß durch Zusatz eines sogn. Stimulators zum Kulturmedium, z.B. eines literaturbekannten Tumorpromotors wie einem Diterpen-Derivat, vorzugsweise vom Tiglian- oder Daphnan-Typ, z.B. Mezerein oder eines Esters des Phorbols, vorzugsweise 12-0-tetradecanoylphorbol-13-acetat (siehe Diamond et al. in Advances in Cancer Research Vol. 32, Seiten 1-74, Academic Press 1980 und Hecker in Carcinogenesis Vol. II, Mechanisms of Tumor Promotion and Cocarcinogenesis, Seiten 11-48, Raven Press, New York 1978), in einer Konzentration von 1-1000 ng/ml die Produktion von Lymphotoxin und von Lymphotoxin-mRNA weiter gesteigert wird.

Als Kulturmedien kommen hierbei die üblichen serumfreien und serumhaltigen Kulturmedien in Betracht, beispielsweise Eagle's Minimum Essential Medium oder Roswell Park Memorial Institute Medium 1640 (RPMI1640), denen jeweils fötales Kalbserum und Antibiotica zugesetzt werden kann, vorzugsweise bis zu 10 % fötales Kalbserum, Penicillin und Streptomycin.

Bei der Produktion der Lymphotoxin-Proteine wird das erfindungsgemäße Verfahren bevorzugt in der Weise durchgeführt, daß transformierte Zellen nach Zusatz von 10 bis 100 ng/ml 12-0-tetradecanoylphorbol-13-acetat oder Mezerein als Stimulator in einem serumhaltigen, unter bestimmten Bedingungen auch in einem serumfreien Kulturmedium, z.B. in RPMI 1640, 1 bis 7 Tage, vorzugsweise jedoch 3 bis 4 Tage gehalten werden und anschließend das so gebildete Lymphotoxin aus dem Kulturüberstand nach an sich bekannten Methoden abgetrennt, konzentriert und gereinigt wird. Besonders vorteilhaft wird dieses Verfahren jedoch in der Weise durchgeführt, daß die Zellen nach anfänglicher Proliferation in einem serumhaltigen Medium in ein serumarmes, vorzugsweise jedoch serumfreies Medium, übergeführt werden.

Bei der Produktion der Lymphotoxin-mRNA wird das erfindungsgemäße Verfahren bevorzugt in der Weise durchgeführt, daß transformierte Zellen nach Zusatz von 10-100 ng/ml 12-0-tetra-decanoylphorbol-13-acetat oder Mezerein als Stimulator in einem serumhaltigen Kulturmedium, z.B. in RPMI1640 und 10 % fötalem Kalbserum 6-48 Stunden, vorzugsweise 12 bis 24 Stunden inkubiert werden, anschließend die Zellen abgetrennt werden und nach Zerstörung dieser die mRNA nach an sich bekannten Verfahren isoliert wird.

Bei der Isolierung der so gebildeten Zellkulturprodukte hat sich bei der Reinigung des so erhaltenen Lymphotoxins die Chromatographie über porenkontrolliertes Glas, welche erstmals angewendet wurde, und, nach Zerstörung der Zellmembranen und Entfernen der Zellkerne, die Extraktion der Lymphotoxin-mRNA beispielsweise mit Phenol als besonders geeignet erwiesen.

Die weitere Reinigung des Lymphotoxins erfolgt beispielsweise mittels HPLC.

Das so erhaltene Lymphotoxin mit einem Molekulargewicht von 60.000 $\pm$ 15 % ist, z.B. in wässriger isotonischer Lösung, zur Prophylaxe und zur Bekämpfung von Tumorzellen geeignet.

Gegenüber dem Stand der Technik weist das erfindungsgemäße Verfahren folgende Vorteile auf:

1. Es wird eine homogene Zellpopulation mit unbegrenzter Lebensfähigkeit verwendet, deren Vermehrung in beliebigem Maßstab erweitert werden kann,

2. die Lymphotoxin-Produktion in diesen Kulturen erfolgt spontan, das heißt es müssen keine hochmolekularen Mitogene zugesetzt werden,

3. die Produktion von Lymphotoxin kann durch niedermolekulare Stimulatoren weiter gesteigert werden,

4. die Produktion von Lymphotoxin kann in serumfreiem Medium mit hohen Ausbeuten erfolgen; man erhält somit ein hervorragendes Ausgangsmaterial für die weitere proteinchemische Reinigung und

5. die aus den Zellen isolierte RNA zeigt unmittelbar nachweisbare Lymphotoxin-mRNA-Aktivität und stellt somit ein hervorragendes Ausgangsmaterial für die weitere Anreicherung dieser mRNA dar, was wiederum eine wesentliche Voraussetzung für die molekulare Klonierung der mRNA darstellt. Die klonierte Affen-Lymphotoxin-mRNA kann wiederum nach bekannten Verfahren als Probe für die Isolierung der homologen humanen Lymphotoxin-mRNA beziehungsweise der entsprechenden Gene verwendet werden.

Die folgenden Beispiele sollen die vorliegende Erfindung näher erläutern, ohne diese jedoch zu beschränken:

## Beispiel 1

### Produktion von Lymphotoxin durch Herpesvirus-transformierte Affen-T-Lymphocyten-Zellinien

Die eingesetzten lymphoiden Zellinien wurden in Roswell Park Memorial Institute Medium 1640 (RPMI 1640) mit Zusatz von 10 % fötalem Kalbserum, Penicillin und Streptomycin in stationärer Kultur bei 37°C in einer Atmosphäre aus 95 % Luft und 5 % Kohlendioxid gehalten und dreimal wöchentlich mit frischem Medium verdünnt. In allen Experimenten wurden dichte Kulturen mit frischem Medium 1:2 bis 1:3 verdünnt, am nächsten Tag nochmals 1:3 verdünnt und jeweils 5 ml in einem Kulturfläschchen angesetzt. Die Kulturüberstände wurden nach drei Tagen durch Zentrifugieren gewonnen, eingefroren und bis zum Test bei -20°C gelagert.

Die nachfolgende Tabelle enthält die in drei verschiedenen Kulturüberständen jeder Zellinie ermittelten Lymphotoxin-Aktivitäten:

| Zellinie | Ursprung (transformierendes Herpesvirus = HV) | Referenz | Lymphotoxinaktivität RE/ml Expt. 1 | Expt. 2 | Expt. 3 |
|---|---|---|---|---|---|
| L-77/5 | Saguinus oedipus - Lymphknoten eines tumortragenden Tieres (HV saimiri) | A | 1 | 3 | 1 |
| A 651 | Saguinus oedipus - in vitro Transformation von Lymphocyten (HV ateles) | B | 15 | 30 | 25 |
| A 2543 | Saguinus oedipus - in vitro Transformation von Lymphocyten (HV ateles) | B | 90 | 56 | 48 |
| 70N2 | Saguinus oedipus - Thymus eines tumortragenden Tieres (HV saimiri) | A,C,D,E | 56 | 47 | 50 |
| 1022 | Saguinus oedipus - Tumor (HV ateles) | D | 580 | 480 | 630 |
| 1670 | Saguinus nigricollis - Milz eines tumortragenden Tieres (HV saimiri) | A,C,E | 88 | 83 | 77 |

Referenzen:

A = Fleckenstein et al., Int. J. Cancer 19, 546-554 (1977)

B = Abb et al., Cancer Immunol. Immunother. 9, 219-226 (1980)

C = Falk et al., Bacteriol. Proc. 38, 191 (1972)

D = Johnson et al., Proc. Natl. Acad. Sci. USA 78, 6391-6395 (1981)

E = Kaschka-Dierich et al., J. Virol. 44, 295-310 (1982)

Übersichtsartikel: Fleckenstein, Biochem. Biophys. Acta 560, 301-342 (1979)

Zur Produktion größerer Mengen von Zellen wurden an Stelle von stationären Kulturen bewegte Kulturen verwendet. Dazu wurden die Zellen (zum Beispiel der Linie 1670) zum Beispiel in RPMI 1640-Medium mit Zusatz von 10 % fötalem Kalbserum in 1- oder 2-Liter Erlenmeyerkolben auf Rotationsschüttlern gezüchtet (0,5 l Kultur pro 1 l-Kolben; 1 l Kultur pro 2 l-Kolben; 40 Umdrehungen pro Minute; 37°C in normaler Atmosphäre) und dreimal wöchentlich mit frischem Kulturmedium im Verhältnis 1:2 bis 1:3 verdünnt. Auf diese Weise ist die Produktion von Kulturüberstand in der Größenordnung von 10-100 l wöchentlich ohne weiteres möglich.

Die Produktion von Lymphotoxin in 1670-Zellen war über lange Zeiträume hinweg stabil: nach 9 Monaten in kontinuierlicher Kultur war keine signifikante Abnahme der Lymphotoxin-Aktivität in den Kulturüberständen feststellbar.

0135797

Charakterisierung des Lymphotoxins:

a) Zum biologischen Nachweis von Lymphotoxin-Aktivität wurde folgende Nachweismethode verwendet (modifiziert nach Trivers et al. in J. Immunol. 117, 130-135 und Evans in Cell. Immunol. 63, 1-15 (1981)):

Transformierte Mauszellen der Zellinie L929 wurden über Nacht mit 1 microCurie 3H-Thymidin/ml Kulturmedium (bevorzugt Eagle's Minimum Essential Medium mit 10% fötalem Kalbserum) kultiviert, dann trypsinisiert und in frischem Kulturmedium mit 5 % fötalem Kalbserum suspendiert. Je 0,5 ml dieser Suspension mit etwa 30.000 Zellen wurden in Kulturschalen (Durchmesser 16 mm) pipettiert und 3-6 Stunden inkubiert. Anschließend wurden serielle Verdünnungen der zu testenden Probe aufgetragen und drei Tage inkubiert (alle Inkubationen bei 37°C). Die Radioaktivität in den Kulturüberständen wurde schließlich im Flüssigszintillationszähler gemessen.

Die nachfolgende Abbildung zeigt den Nachweis der Lymphotoxin-Aktivität eines Kulturüberstandes von 1670-Zellen. Jede Verdünnung wurde parallel in 4 Kulturschalen getestet. Nur die Mittelwerte wurden angegeben, die Standardabweichung betrug bei den ersten drei Verdünnungen 3-4 %, bei der höchsten Verdünnung 6 %. Der in diesem Experiment verwendete Kulturüberstand wurde in mehreren hundert Ampullen eingefroren und als Referenzstandard in allen Lymphotoxintests mitgeführt; seine Aktivität wurde willkürlich mit 100 Referenz-Einheiten pro Milliliter (RE/ml) angenommen:

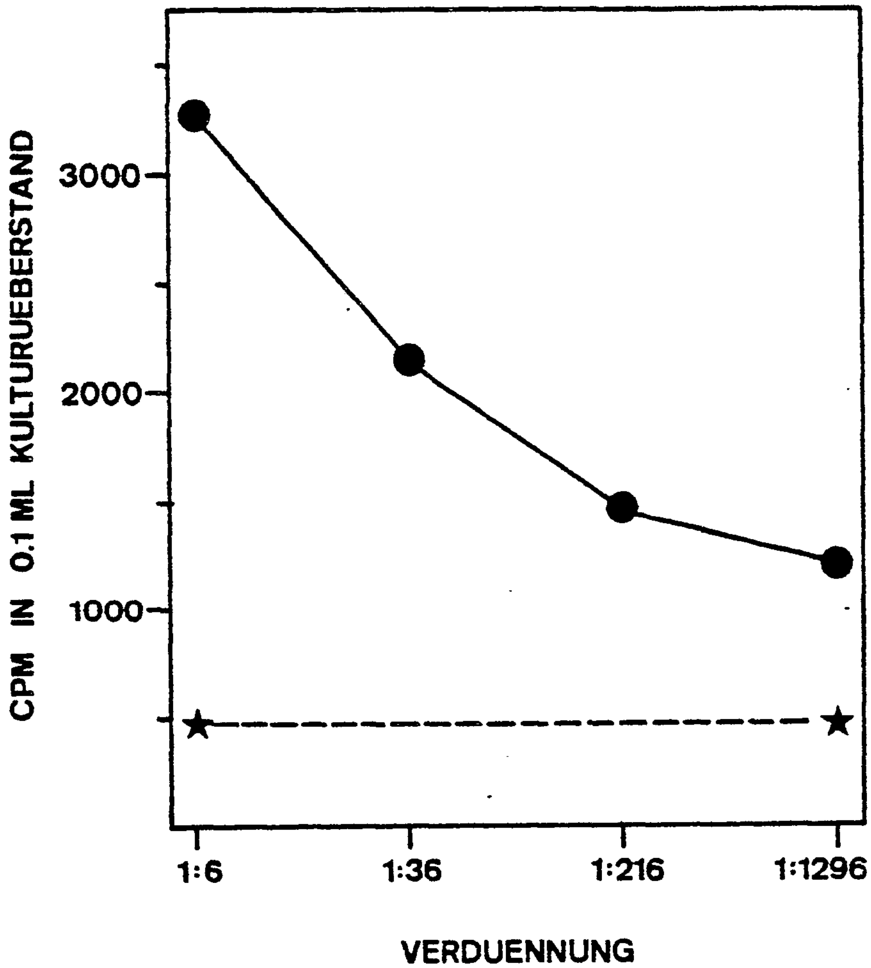


● Kulturüberstand von 1670-Zellen
✱ Kontrollen (Medium)

Zum Vergleich wurden Kulturüberstände einer Reihe von menschlichen T-Lymphocyten-Zellinien (MOLT-4, 1301, JURKAT, Karpas-45, CCRF-CEM und CCRF-HSB-2) in dem oben angeführten biologischen Lymphotoxin-Test untersucht. In keinem Fall konnte eine signifikante Steigerung der 3H-Thymidin-Freisetzung gegenüber unbehandelten Kontrollen nachgewiesen werden.

b) Die physikalisch-chemische Charakterisierung der Lymphotoxin-Aktivität in Kulturüberständen der Zellinie 1670 wurde durch folgende Experimente vorgenommen:

Stabilität gegenüber thermischer Belastung
Stabilität gegenüber saurem pH-Wert
Stabilität gegenüber Frieren/Tauen
Stabilität gegenüber Natriumdodecylsulfat (SDS)
Stabilität gegenüber reduzierenden Agentien (2-Mercaptoethanol)
Molekulargewichtsbestimmung durch Hochdruckflüssigkeitschromatographie (HPLC)

Die folgende Tabelle zeigt die Ergebnisse der Stabilitätsprüfungen:

| Behandlung | Lymphotoxin-Aktivität in Prozent des Kontrollwertes |
|---|---|
| 56°C - 5 Stunden | 68 % |
| 5 Zyklen Frieren/Tauen | 108 % |
| pH 2.0 - 24 Stunden bei 4°C | 1 % |
| 0,1 % SDS - 1 Stunde bei 37°C | 15 % |
| 0,1 M 2-Mercaptoethanol - - 1 Stunde bei 37°C | 95 % |

Die Aktivität ist sehr stabil gegenüber Mercaptoethanol, gegenüber Erhitzen und mehrere Zyklen von Frieren/Tauen, und wird bei pH 2 innerhalb von 24 Stunden vollkommen zerstört.

Die Experimente zur thermischen und pH-Stabilität sowie gegenüber Frieren/Tauen wurden mit dem Standard-Kulturüberstand von unbehandelten 1670-Zellen in Gegenwart von 10 % fötalem Kalbsserum durchgeführt. Die anderen beiden Experimente wurden mit einem über porenkontrolliertes Glas (siehe Beispiel 4) konzentrierten und angereicherten Kulturüberstand von mit Mezerein (20 ng/ml; siehe Beispiel 4) behandelten 1670-Zellen durchgeführt (Proteingehalt 1-2 mg/ml), der nach der Inkubation mit SDS beziehungsweise Mercaptoethanol mit kompletten Kulturmedium (10 % Kalbsserum) 50fach verdünnt wurde.

Das Molekulargewicht des Lymphotoxins aus 1670-Zellen wurde durch Hochdruck-Flüssigkeits-Chromatographie bestimmt (HPLC - siehe nachfolgende Abbildung):

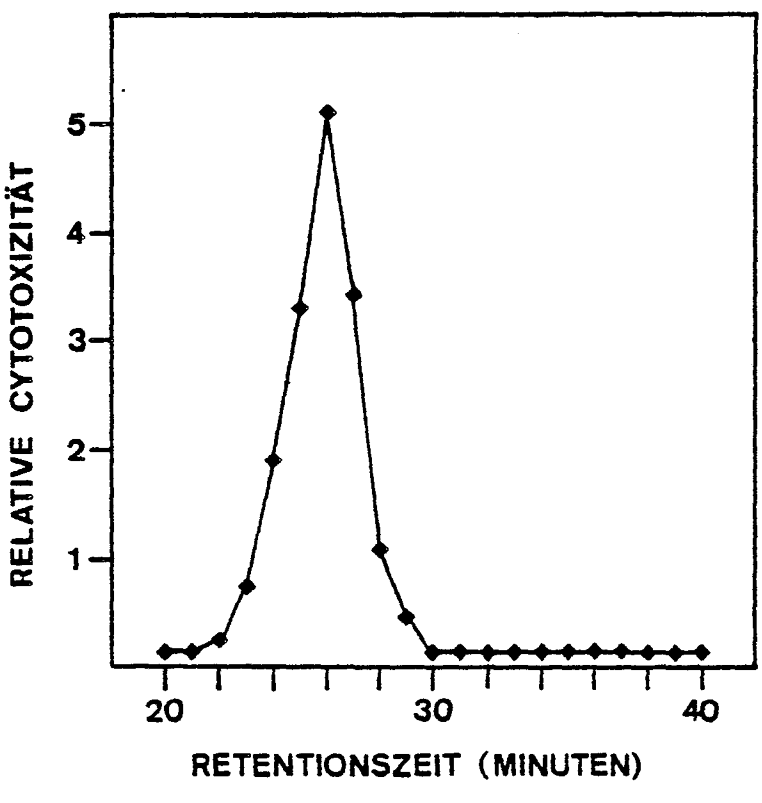


200 microliter eines über porenkontrolliertes Glas konzentrierten Kulturüberstandes unbehandelter 1670-Zellen wurden mittels HPLC auf einer Anlage der Fa. WATERS aufgetrennt (2 Säulen I-125; 20 mM Natriumphosphat pH 7, 1 M NaCl, 0,1 % Tween 20 (Polyoxyethylen-sorbitan-monolaureat,n = 20, M.G.: etwa 1200), 25 % (v/v) Propylenglykol; 0,5 ml pro Minute).

0,5 ml - Fraktionen wurden aufgefangen und getestet. Als Eichproteine zur Bestimmung des Molekulargewichts wurden Serumalbumin, Ovalbumin, Trypsinogen und Lysozym im selben System aufgetrennt. Die Analyse zeigte einen einzigen Aktivitätspeak bei einem Molekulargewicht von 60.000 ± 15 %.

c) Die wachstumshemmende Wirkung von Kulturüberständen der Zellinie 1670 wurde an einer Reihe von transformierten (Tumor-) Zellinien untersucht. In diesen Versuchen wurden die Tumorzellen (kultiviert in Eagle' s Minimum Essential Medium mit 10 % fötalem Kalbserum, Penicillin und Streptomycin) in Kulturschalen angesetzt (50.000 Zellen pro Schale mit 3 cm Durchmesser) und einige Stunden später mit Medium oder dem Standard-Kulturüberstand der Zellinie 1670 versetzt (Endkonzentration 16 RE/ml; 3 ml/Schale). Nach 3 oder 4 Tagen Inkubation bei 37°C wurde die Zellzahl pro Schale bestimmt (in allen Experimenten wurden jeweils zwei Schalen parallel mit Lymphotoxin bzw. Medium angesetzt). Bei drei Zellinien wurde die Zellzahl täglich bestimmt. Die Ergebnisse sind in der folgenden Tabelle bzw. Abbildung dargestellt:

| Spezies | Zellinie | Inkubationsdauer (Tage) | Zellzahl /3 cm-Kulturschale (10E-5 x Zellen/Schale) Kontrolle | Lymphotoxin |
|---|---|---|---|---|
| Mensch | Hela | 4 | 8,1 | 0,6 |
| | Hep-2 | 3 | 9,3 | 2,1 |
| | A-549 | 4 | 10 | 3,4 |
| Affe | GL-V3 | 3 | 5,6 | 4,5 |
| | Vero | 3 | 6,3 | 5,3 |
| Maus | L929 | 4 | 8,5 | 3,1 |
| | IT22 | 3 | 13 | 4,0 |
| | B16 | 4 | 19 | 4,9 |
| Kaninchen | RK13 | 3 | 4,6 | 3,5 |

0135797

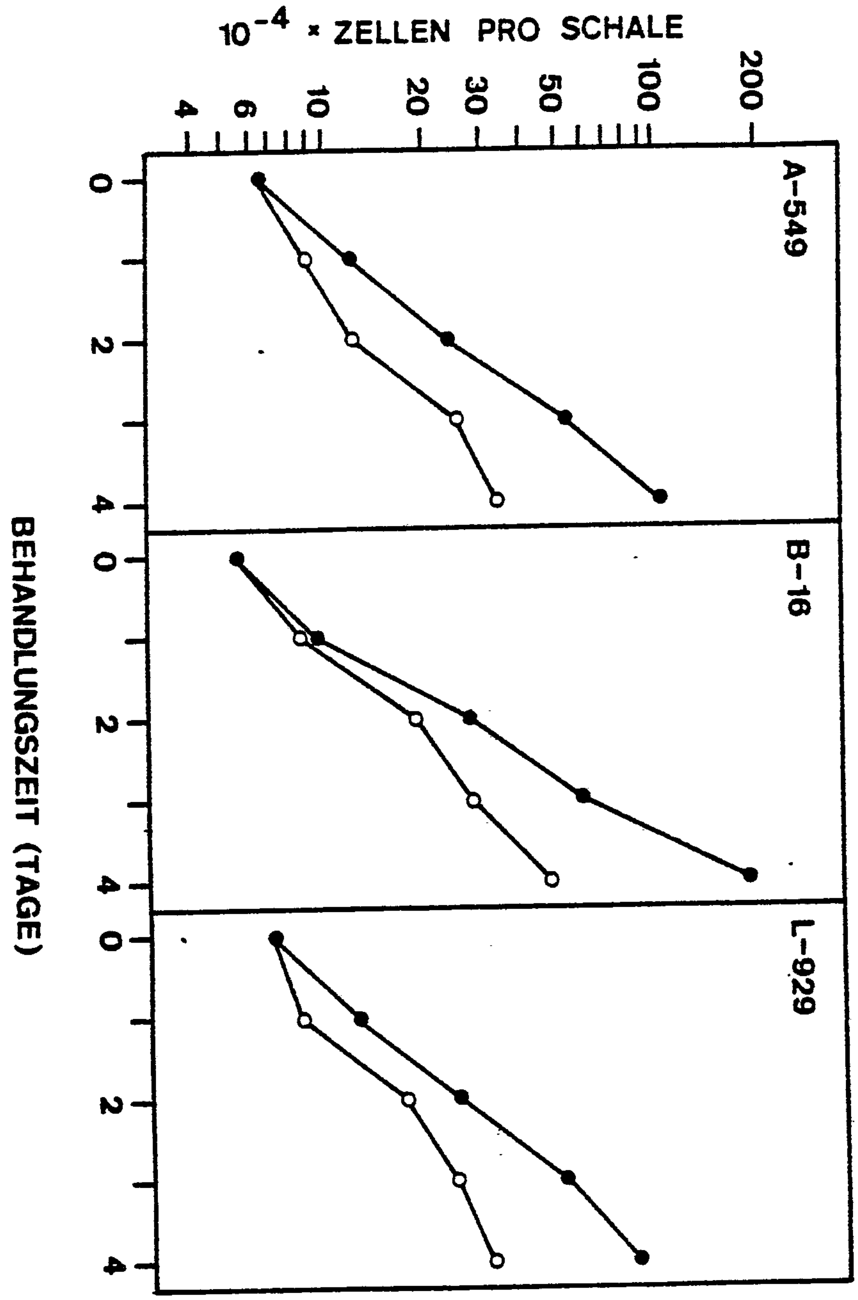


● Kontrollen

○ Lymphotoxin

Diese zeigen, daß Kulturüberstände aus 1670-Zellen die Vermehrung von Tumorzellen verschiedener Spezies hemmen können. Diese Eigenschaft ist charakteristisch für Lymphotoxine (Evans in Cancer Immunol. Immunother. 12, 181-190 (1983)); sie unterscheidet diese Gruppe von Proteinen insbesondere von den Interferonen, die ebenfalls das Wachstum von Tumorzellen hemmen können, im allgemeinen jedoch spezies-spezifisch sind, wobei insbesondere Humaninterferon auf Mauszellen sehr wenig oder überhaupt nicht aktiv sind.

Die vorstehend erwähnten transformierten menschlichen T-Lymphocyten-Zellinien sowie die (Tumor-)Zellinien sind literaturbekannt.

Die Zellinie 1670 wurde am 28. März 1984 unter der Nummer I-294 bei der "Collection nationale de cultures de microorganismes (C.N.C.M.), Institut Pasteur, Paris" gemäß Regel 28 des Europäischen Patentübereinkommens hinterlegt.

## Beispiel 2

### Steigerung der Lymphotoxin-Produktion in Affen-T-Lymphocyten-Zellinien durch Diterpen-Verbindungen

Dichte Kulturen wurden mit frischem Medium 1:2 bis 1:3 verdünnt, am nächsten Tag nochmals 1:3 verdünnt und jeweils 5 ml derselben Stammkultur in drei Fläschchen aufgeteilt. Die Stimulatoren wurden zugegeben (Stammlösungen 100 microgramm/ml in Dimethylsulfoxid) und die Kulturen wurden drei Tage inkubiert. Anschließend wurden die Zellen durch Zentrifugation entfernt, die Überstände eingefroren und bei -20°C bis zum Lymphotoxintest aufbewahrt.

Die folgende Tabelle zeigt die Wirkung von zwei ausgewählten Diterpen-Tumorpromotoren, nämlich 12-0-tetradecanoyl-phorbol-13-acetat (TPA) und Mezerein, auf die Lymphotoxin-Produktion in ausgewählten Affen-T-Lymphocyten-Zellinien:

| Zellinie | Experiment | Konzentration (ng/ml) | Lymphotoxin-Aktivität im Überstand (RE/ml) Kontrolle | TPA | Mezerein |
|---|---|---|---|---|---|
| 1670 | 1 | 20 | 83 | 520 | 270 |
| | 2 | 20 | 77 | 210 | 76 |
| | 3 | 100 | 88 | 340 | 370 |
| 70N2 | 1 | 20 | 56 | 310 | 280 |
| | 2 | 20 | 47 | 120 | 240 |
| | 3 | 100 | 50 | 65 | 92 |
| | 4 | 100 | 12 | 130 | 160 |
| A651 | 1 | 20 | 260 | 360 | 370 |
| | 2 | 100 | 130 | 320 | 270 |
| A2543 | 1 | 20 | 56 | 140 | 190 |
| | 2 | 100 | 48 | 190 | 180 |
| L77/5 | 1 | 20 | 3 | 36 | 86 |
| | 2 | 100 | 1 | 38 | 35 |
| 1022 | 1 | 100 | 480 | 760 | 930 |
| | 2 | 100 | 630 | 700 | 610 |

Beispiel 3

Produktion von Lymphotoxin in serumfreiem Medium

Nach der Proliferation der Zellen in serumhaltigem Medium (analog Beispiel 2) wurden die Zellen in serumfreies Medium übergeführt.

Die nachfolgende Tabelle zeigt einen Vergleich der Lymphotoxin-Produktion in 1670-Zellen analog Beispiel 1 jeweils in serumhältigen und serumfreien Medium in Gegenwart von 20 ng Mezerein/ml:

| Experiment | Serumgehalt des Mediums ( % ) | Lymphotoxin-Aktivität (RE/ml) | Proteingehalt (mg/ml) | Spez. Aktivität (RE/mg) |
|---|---|---|---|---|
| 1 | 10 | 370 | 5 | 74 |
| 2 | 10 | 180 | 5 | 36 |
| 3 | 10 | 270 | 5 | 54 |
| 4 | 0 | 140 | 0.050 | 2.800 |
| 5 | 0 | 72 | 0.053 | 1.400 |
| 6 | 0 | 160 | 0.044 | 3.700 |

Die Proteinbestimmung wurde nach der Methode von Bradford (siehe Anal. Biochem. 72, 298 (1976)) durchgeführt.

Beispiel 4

Konzentration und Reinigung von Lymphotoxin aus 1670-Zellen durch Chromatographie über Säulen aus porenkontrolliertem Glas

1670-Zellen wurden in RPMI-1640 Medium mit 10 % fötalem Kalbserum in rotierenden Erlenmeyerkolben gezüchtet (0.5 l Kultur in einem 1 l-Kolben, Rotationsgeschwindigkeit 40 Umdrehungen pro Minute). Zellen aus gut wachsenden Kulturen wurden durch Zentrifugation gewonnen, gewaschen und in serumfreiem RPMI-1640-Medium mit Zusatz von 20 ng/ml Mezerein resuspendiert. Die Suspension wurde in 150 $cm^2$-Plastikkulturflaschen (200 ml pro Flasche) drei Tage lang stationär gehalten, anschließend wurde der Kulturüberstand durch Zentrifugation und Filtration gewonnen.

Derartig hergestellte Kulturüberstände wurden über 10 ml porenkontrolliertes Glas chromatographiert (Porendurchmesser: 350 Angstroem, Korngröße: 120-200 mesh; Säulendurchmesser: 9 mm, Flußgeschwindigkeit: 140 ml/Stunde). Anschließend wurde die Säule mit 10 mM Natrium-Phosphatpuffer pH 7.2/1.5 M NaCl gewaschen und schließlich mit dem gleichen Puffer in 50 % Ethylenglykol (v/v) eluiert.

Die folgende Tabelle gibt die Ergebnisse von zwei derartigen Experimenten wieder:

| | Volumen (ml) | Lymphotoxin (RE/ml) | % | Protein (mg/ml) | spezifische Aktivität RE/mg |
|---|---|---|---|---|---|
| Experiment 1 | | | | | |
| Probe | 2.800 | 162 | 100 | 0.044 | 3.700 |
| Durchlauf | 2.800 | 13 | 8 | - | - |
| Waschfraktion | 10 | 96 | 1 | - | - |
| Eluat | 20 | 17.300 | 76 | 2.0 | 8.650 |
| Experiment 2 | | | | | |
| Probe | 2.500 | 141 | 100 | 0.050 | 2.800 |
| Eluat | 17 | 8.600 | 41 | 1.6 | 5.400 |

Beispiel 5

Nachweis von Lymphotoxin-mRNA-Aktivität in RNA-Präparationen aus 1670- und 1022-Zellen

Die Zellen wurden in 800 ml Zellkulturmedium mit 10 % fötalem Kalbserum bei einer Zelldichte von etwa 5 x $10^5$ Zellen/ml suspendiert und mit 20 ng Mezerein/ml versetzt. 20 Stunden später wurden die Zellen durch Zentrifugation geerntet, in einem geeigneten Puffer (zum Beispiel 10 mM Tris.HCl pH 7.4, 140 mM NaCl, 1,5 mM $MgCl_2$) gewaschen und in 10 ml Lyse-Puffer (zum Beispiel 10 mM Tris.HCl pH 8.4, 140 mM NaCl, 1,5 mM $MgCl_2$, 0.5 % Nonidet-P 40 [nichtionisches Detergens, Warenzeichen der Firma SHELL] suspendiert. Nach zehnminütiger Inkubation im Eisbad wurde die Suspension kurz geschüttelt und die Zellkerne durch Zentrifugation entfernt. Aus dem Überstand der Zentrifugation (= Cytoplasma) wurde durch an sich bekannte Verfahren, zum Beispiel durch Extraktion mit Phenol (siehe Aviv et al. in Proc. Natl. Acad. Sci. USA 69, 1408-1412 (1977)) die RNA isoliert. Sie wurde schließlich in Wasser gelöst (Konzentration etwa 3-5 mg/ml) und mittels an sich bereits bekannter Verfahren in Xenopus laevis - Oocyten translatiert (siehe beispielsweise Colman et al. in Cell 17, 517-526 (1979), Hitchcock et al. in Anal. Biochem. 109, 338-344 (1980) und Contreras et al. in Anal. Biochem. 113, 185-187 (1981)). Die Translationsprodukte wurden im biologischen Test auf ihre Lymphotoxin-Aktivität überprüft.

Die folgende Tabelle zeigt die aufgefundene cytotoxische Aktivität in Oocytenüberständen im Vergleich zu RNA-Präparationen aus MOLT-4 Zellen, einer humanen T-Lymphocytenzellinie, deren Kulturüberstände keine nachweisbare Lymphotoxin-Aktivität aufweisen, sowie Überständen von unbehandelten Oocyten:

| Präparat | | Radioaktivität im Überstand (cpm/100 microliter) Verdünnung der Oocyten - Überstände | | | |
|---|---|---|---|---|---|
| | | 1 : 12 | 1 : 42 | 1 : 147 | 1 : 514 |
| Kontrollen (nicht injizierte Oocyten) | | 2.200 | 2.300 | 2.100 | 2.500 |
| | | 2.400 | 2.200 | 1.800 | 3.000 |
| MOLT-4 RNA | Expt. 1 | 1.600 | 2.400 | 1.700 | 2.200 |
| | Expt. 2 | 1.700 | 2.300 | 1.600 | 1.800 |
| 1670-RNA | Expt. 1 | 4.700 | 4.700 | 3.600 | 2.800 |
| | Expt. 2 | 4.300 | 3.800 | 3.300 | 2.600 |
| | Expt. 3 | 3.200 | 2.800 | 2.800 | |
| Kontrollen | | 4.400 | 3.300 | - | - |
| 1670-RNA | Expt. 1 | 6.100 | 5.000 | - | - |
| 1670-RNA | Expt. 2 | 6.600 | 5.100 | - | - |
| 1022-RNA | Expt. 1 | 7.000 | 5.000 | - | - |

Die Tabelle zeigt, daß alle drei RNA-Präparationen aus 1670-Zellen sowie eine RNA-Präparation aus 1022-Zellen in bis zu 147-facher Verdünnung eine signifikante Steigerung der freigesetzten Radioaktivität bewirkten, während RNA aus MOLT-4-Zellen keine derartige Aktivität aufweist. Diese Experimente zeigen, daß in RNA-Präparationen aus 1670- und 1022-Zellen biologisch aktive Lymphotoxin-mRNA enthalten ist.

## PATENTANSPRÜCHE

1. Verfahren zur Herstellung von Lymphotoxin oder Lymphotoxin-mRNA, dadurch gekennzeichnet, daß mit Viren transformierte Affen-T-Lymphocyten in einem geeigneten Medium in permanenter Kultur gezüchtet werden und das Lymphotoxin oder die Lymphotoxin-mRNA nach an sich bekannten Methoden isoliert wird.

2. Verfahren nach Anspruch 1, dadurch gekennzeichnet, daß als Viren Herpesviren, vorzugsweise jedoch Herpesvirus saimiri oder Herpesvirus ateles, verwendet werden.

3. Verfahren nach Anspruch 1, dadurch gekennzeichnet, daß als Affen-T-Lymphocyten Zellen aus der Gattung Saguinus verwendet werden.

4. Verfahren nach Anspruch 3, dadurch gekennzeichnet, daß als T-Lymphocyten-Zellinien die Linien 1022, 1670, 70N2, L77/5, A651 oder A' 2543 verwendet werden.

5. Verfahren nach Anspruch 3, dadurch gekennzeichnet, daß als T-Lymphocyten-Zellinien die Linien 1022 oder 1670 verwendet werden.

6. Verfahren nach den Ansprüchen 1-5, dadurch gekennzeichnet, daß dem Kulturmedium bis zu 10 % fötales Kalbserum zugesetzt wird.

7. Verfahren nach den Ansprüchen 1-6, dadurch gekennzeichnet, daß dem Kulturmedium zur Produktionsstimulation niedermolekulare Tumorpromotoren, vorzugsweise Diterpene, insbesondere vom Daphnan-, Ingelan- und Tigliantyp, oder Indolalkaloide zugesetzt werden.

8. Verfahren nach den Ansprüchen 1 bis 7, dadurch gekennzeichnet, daß zur Stimulation der Produktion von Lymphotoxin während der Inkubation als Tumorpromotor ein Ester des Phorbols oder Mezerein, in einer Konzentration von 1-1000 ng/ml, vorzugsweise jedoch von 10-100 ng/ml, zugesetzt wird.

9. Verfahren nach den Ansprüchen 1 bis 7, dadurch gekennzeichnet, daß zur Stimulation der Produktion von Lymphotoxin-mRNA während der Inkubation als Tumorpromotor ein Ester des Phorbols oder Mezerein, in einer Konzentration von 1-1000 ng/ml, vorzugsweise jedoch von 10-100 ng/ml, zugesetzt wird.

10. Verfahren nach den Ansprüchen 1 bis 8, dadurch gekennzeichnet, daß nach Proliferation der Zellen in serumhaltigem Medium die Zellen in serumfreies Medium überführt werden.

11. Verfahren nach den Ansprüchen 1 bis 8 und 10, dadurch gekennzeichnet, daß das Lymphotoxin aus den Kulturüberständen nach 1 bis 7 Tagen, bevorzugt aber nach 2 bis 4 Tagen, abgetrennt wird.

12. Verfahren nach den Ansprüchen 1 bis 8, 10 und 11, dadurch gekennzeichnet, daß das Lymphotoxin über eine Säule aus porenkontrolliertem Glas angereichert wird.

13. Verfahren nach den Ansprüchen 1 bis 7 und 9, dadurch gekennzeichnet, daß die Zellen in einem Kulturmedium, das fötales Kalbsserum enthält, inkubiert werden und die Lymphotoxin-mRNA aus den Zellen nach 3 bis 48 Stunden, bevorzugt aber nach 6 bis 24 Stunden, isoliert wird.

14. Lymphotoxin, herstellbar nach den Ansprüchen 1 bis 8 und 10 bis 12.

15. Lymphotoxin nach Anspruch 14, dadurch gekennzeichnet, daß dieses ein Molekulargewicht von 60.000 ± 15 % aufweist.

16. Lymphotoxin-mRNA, herstellbar nach den Ansprüchen 1 bis 7, 9 und 13.

17. Arzneimittel, enthaltend Lymphotoxin gemäß den Ansprüchen 14 oder 15.

18. Arzneimittel gemäß Anspruch 17 zur Bekämpfung von Tumorzellen.

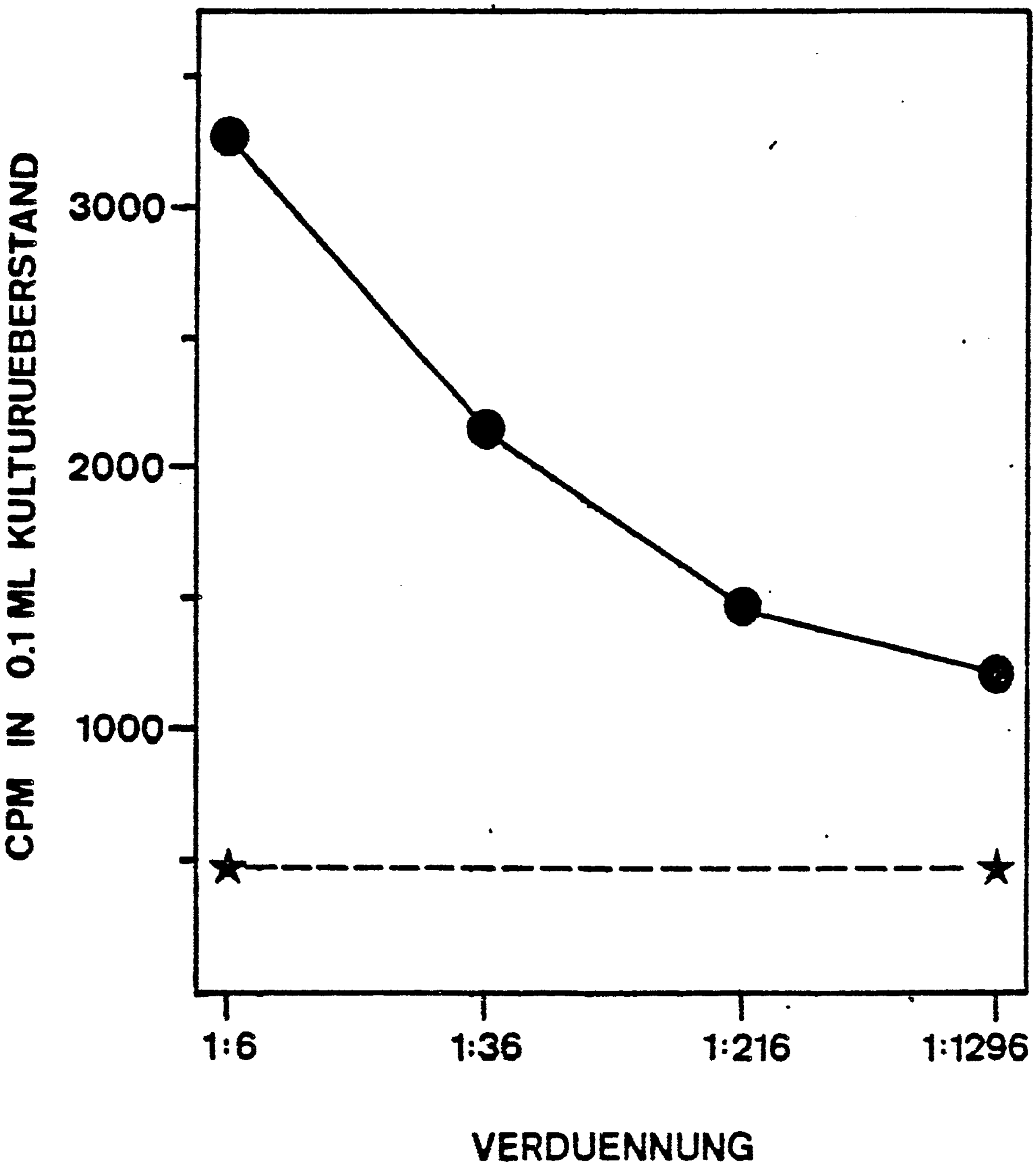
CPM IN 0.1 ML KULTURUEBERSTAND
3000
2000
1000
1:6
1:36
1:216
1:1296
VERDUENNUNG

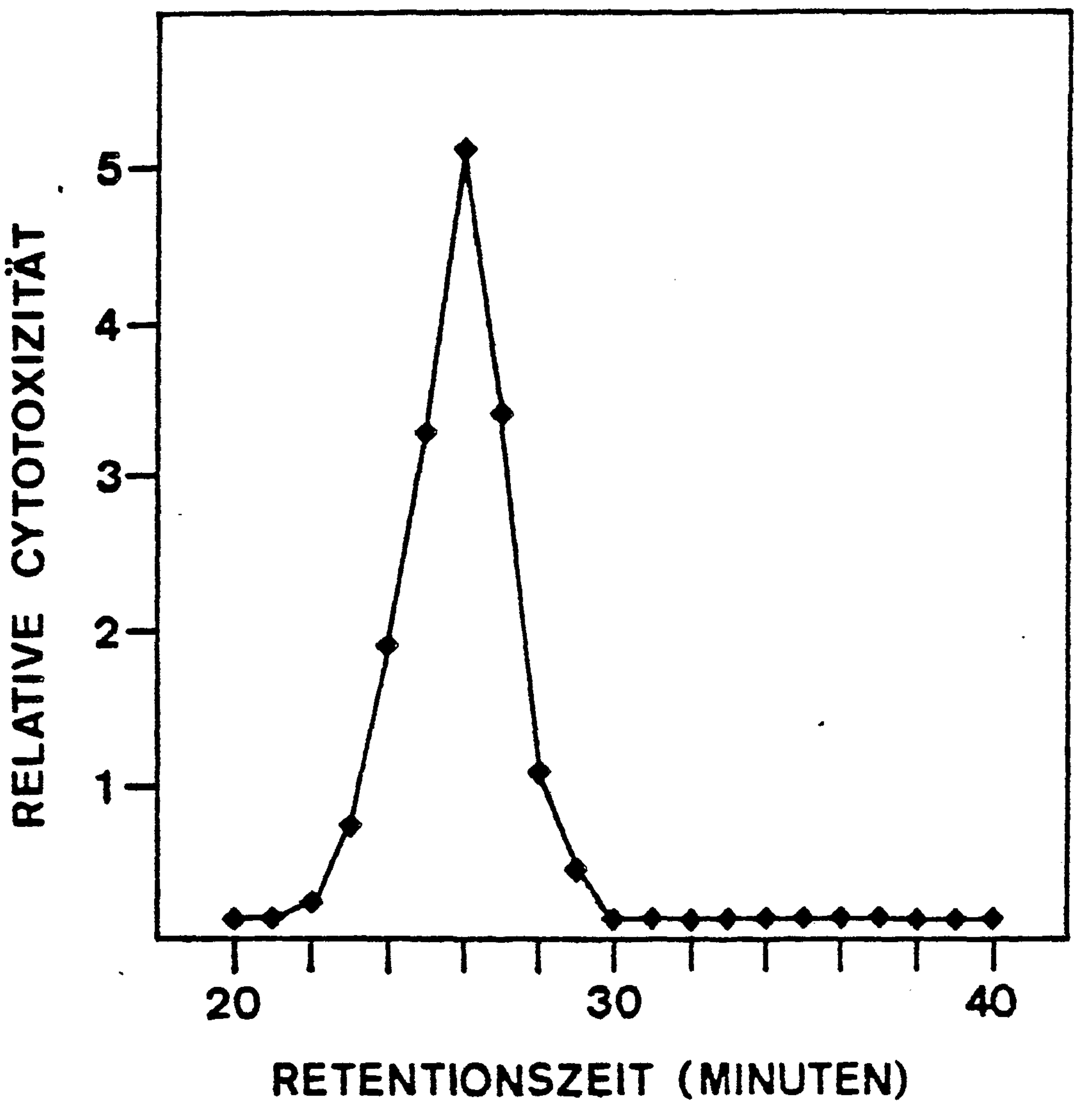

RELATIVE CYTOTOXIZITÄT
5
4
3
2
1
20
30
40
RETENTIONSZEIT (MINUTEN)

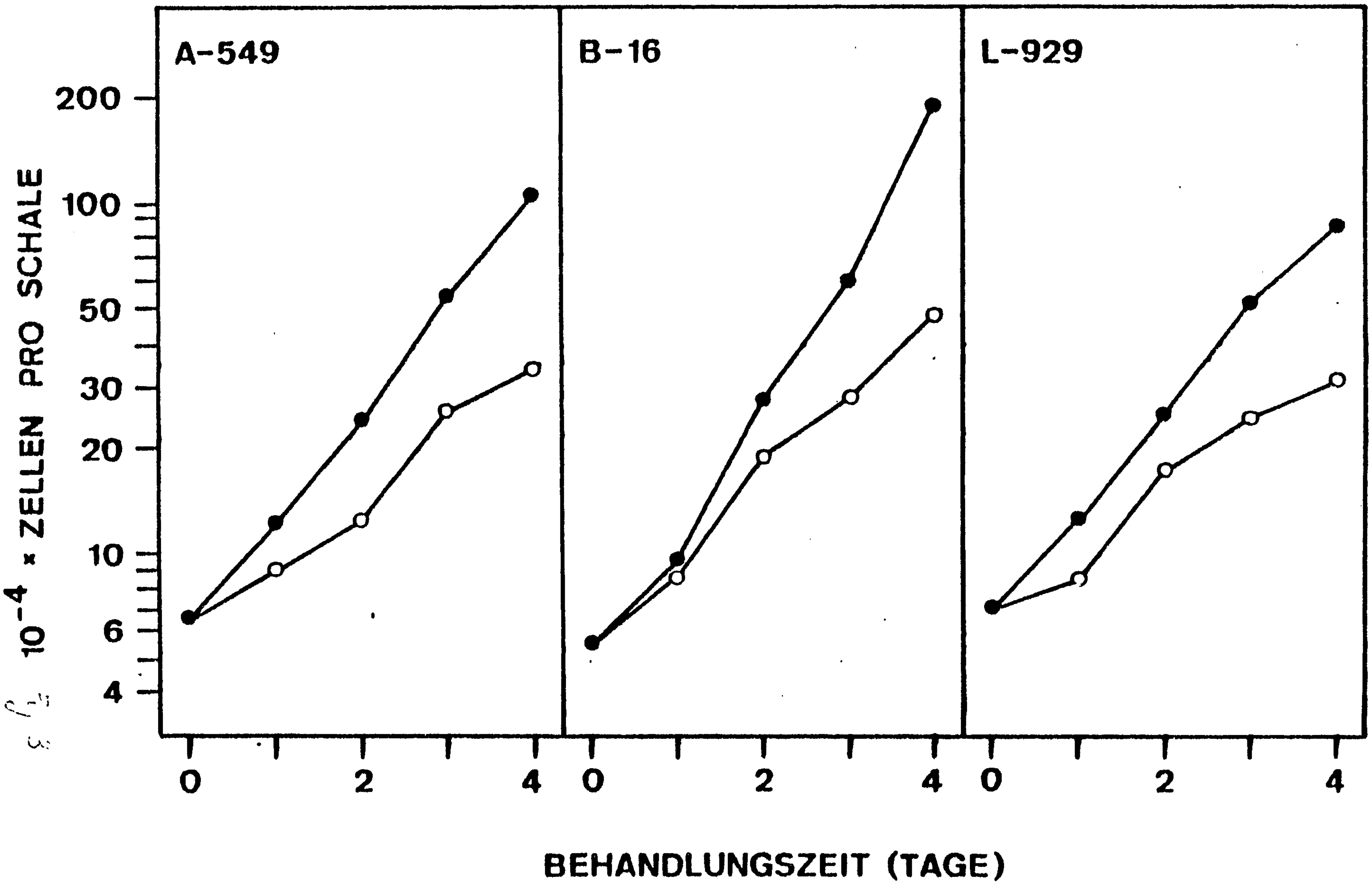
A-549
B-16
L-929
200
100
50
30
20
10
6
4
$10^{-4}$ × ZELLEN PRO SCHALE
0
2
4
0
2
4
0
2
4
BEHANDLUNGSZEIT (TAGE)